# EUROPEAN PATENT SPECIFICATION

(11) **EP 3 694 431 B1**
(45) Date of publication and mention of the grant of the patent: **18.11.2026**
(21) Application number: 18797210.4
(22) Date of filing: 28.09.2018
(51) Int. Cl.: A61B 17/86

(54) **EASY START CANNULATED BONE SCREW**
KANÜLIERTE KNOCHENSCHRAUBE MIT LEICHTEM START
VIS À OS CANULÉE À DÉMARRAGE FACILE

(30) Priority: 09.10.2017 US 201762569770 P; 15.12.2017 US 201762599228 P
(43) Date of publication of application: 19.08.2020
(73) Proprietor: CONMED Corporation, Largo, FL 33773 (US)
(72) Inventor: MUSER, Andrew P., St. Pete Beach, FL 33706 (US)
(74) Representative: Strehl & Partner mbB
(86) International application number: PCT/US2018/053412
(87) International publication number: WO 2019/074696

(56) References cited:
- US-A1- 2012 136 398
- US-A1- 2013 231 708
- US-A1- 2013 253 594
- US-A1- 2016 310 187

## Description

### BACKGROUND OF THE INVENTION

### 1. Field of the Invention

The present disclosure is directed generally to a surgical fixation device and, more particularly, to a cannulated screw for fixation into bone or soft tissue.

### 2. Description of Related Art

In orthopedic surgeries, screws are often used to secure and fix biological material or another object, such as an implant, in a desired position relative to a bone. For example, screws are used to attach a bone block to an existing bone or to secure soft tissue to a bone or soft tissue. The initial threading of the screw into the bone or a bone block can be difficult. Therefore, in some procedures, a pilot hole is required to aid in alignment of the screw. As well understood in the surgical field, the pilot hole is smaller in depth and diameter than the screw to provide a guide for the screw, while still being narrow enough to allow enough of the threads of the screw to penetrate the boney interference site. A mallet may also be used to hammer the screw deep enough for the threads to penetrate the boney interference site. US 2013/0253594 A1 discloses a surgical screw with the features in the preamble of present claim 1. Another conventional screw is defined in US 2016/0310187 A1.

Drilling a pilot hole requires additional surgical time and additional surgical instruments. While using a mallet to hammer the screw into the bone may be less time consuming and require less instrumentation, it is less precise. Hammering the screw into the bone carries a greater risk that the screw will become misaligned with the desired fixation location. Therefore, there is a need for a surgical screw that does not require the use of a pilot hole or a mallet, while maintaining sturdy and reliable fixation within the bone.

### SUMMARY OF THE INVENTION

The present invention is directed to a surgical screw as defined in claim 1. The surgical screw comprises features for reducing the torque required to start or introduce the screw into the anatomy. According to one aspect, the surgical screw includes a cannulated body having a proximal end and a distal end. The distal end of the cannulated body can have a tapered tip, and a plurality of threads which can extend around the exterior of the cannulated body from a position at the very distal end, a position adjacent to the distal end, or a position proximal to the distal end to a position at the very proximal end, a position adjacent to the distal end, or a position distal of the proximal end. An introducer extends distally from the tapered tip and has a constant diameter which is less than a diameter of the tapered tip. The surgical screw is cannulated such that a channel extends through the body from the proximal end to the distal end.

According to another aspect, the surgical screw includes a cannulated body having a proximal end and a distal end, which has a tapered tip. A plurality of threads extend around the exterior of the cannulated body (from positions as described above). The plurality of threads include a primary thread and a secondary thread. A lead of the primary thread is greater than a pitch of the plurality of threads. An introducer extends distally from the tapered tip. The surgical screw is cannulated such that a channel extends through the body from the proximal end to the distal end.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

One or more aspects of the present invention are particularly pointed out and distinctly claimed as examples in the claims at the conclusion of the specification. The foregoing and other objects, features, and advantages of the invention are apparent from the following description taken in conjunction with the accompanying drawings in which:
FIG. 1 is a proximal perspective view schematic representation of a surgical screw, according to an embodiment;
FIG. 2 is a distal perspective view schematic representation of a surgical screw, according to an embodiment;
FIG. 3 is side view schematic representation of a surgical screw, according to an embodiment;
FIG. 4 is a perspective view schematic representation of a surgical screw, according to an alternative embodiment;
FIG. 5 is a side view schematic representation of a surgical screw, according to an alternative embodiment;
FIG. 6 is a distal perspective view schematic representation of a surgical screw, according to an alternative embodiment;
FIG. 7 is a distal end view schematic representation of a surgical screw, according to an alternative embodiment;
FIG. 8 is a distal perspective view schematic representation of a surgical screw, according to an embodiment;
FIG. 9 is a side view schematic representation of a surgical screw, according to an alternative example that does not fall within the scope of the invention as claimed; and
FIG. 10 is a side view schematic representation of a surgical screw, according to another example that does not fall within the scope of the invention as claimed;
FIG. 11 is a side view schematic representation of a surgical screw, according to yet another example that does not fall within the scope of the invention as claimed;
FIG. 12 is a close-up view schematic representation of a surgical screw with a single start; and
FIG. 13 is a close-up view schematic representation of an exemplary embodiment of a surgical screw with a double start.

### DETAILED DESCRIPTION OF THE INVENTION

Aspects of the present invention and certain features, advantages, and details thereof, are explained more fully below with reference to the non-limiting examples illustrated in the accompanying drawings. Descriptions of well-known structures are omitted so as not to unnecessarily obscure the invention in detail. It should be understood, however, that the detailed description and the specific non-limiting examples, while indicating aspects of the invention, are given by way of illustration only, and are not by way of limitation. Various substitutions, modifications, additions, and/or arrangements, within the scope of the underlying inventive concepts will be apparent to those skilled in the art from this disclosure. The scope of the invention is defined by the appended claims.

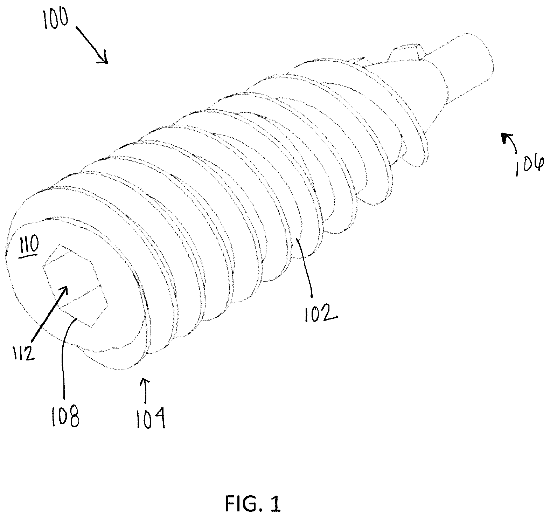

Referring now to the figures, wherein like reference numerals refer to like parts throughout, FIG. 1 shows a proximal perspective view schematic representation of a surgical screw 100, according to an embodiment. The surgical screw 100 comprises a body 102 extending between a proximal end 104 and a distal end 106. The surgical screw 100 can be composed of any suitable biocompatible material, such as titanium or magnesium, and can be manufactured according to common manufacturing methods, such as machining (as should be understood by a person of ordinary skill in the art in conjunction with a review of this disclosure). The surgical screw 100 may also be composed of bio-composite material and manufactured via injection molding (as should be understood by a person of ordinary skill in the art in conjunction with a review of this disclosure).

As shown in FIG. 1, the body 102 comprises a feature 108 at the proximal end 104 that allows the surgical screw 100 to be torqued. Specifically, the feature 108 is on an outer surface 110 of the proximal end 104. For example, the feature 108 can be that of any conventional screw head and non-conventional geometries as well (e.g., Tri-Lobe driver geometry). In the depicted embodiment, the feature 108 has a hexagonal cross-section. As shown in FIG. 1, the surgical screw 100 is cannulated such that a channel 112 extends from the feature 108 at the outer surface 110 of the proximal end 104 through the body 102 to the distal end 106.

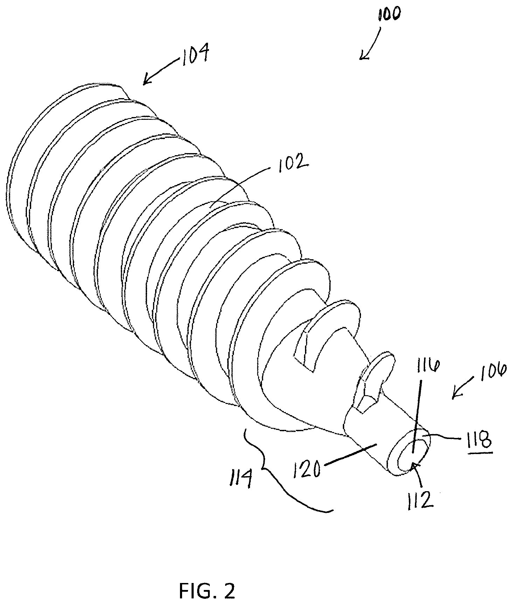

Turning now to FIG. 2, there is shown a distal perspective view schematic representation of the surgical screw 100, according to an embodiment. In the depicted embodiment, the body 102 of the surgical screw 100 comprises a tip 114 at the distal end 106. In FIG. 2, the tip 114 is a tapered region (at least partially tapered, can be tapered all the way to the very distal end, and can include a constant, variable - increasing or decreasing taper, or more than one taper (which may be separated with a section with no taper) with the same or different taper value) such that the body 102 is tapered toward the distal end 106. As described above, the surgical screw 100 is cannulated. FIG. 2 shows the channel 112 extending through the body 102 of the surgical screw 100 to the tip 114 at the distal end 106. Specifically, the channel 112 extends from the feature 108 at the proximal end 104 to an opening 116 on an outer surface 118 of the tip 114.

Still referring to FIG. 2, the surgical screw 100 comprises an introducer 120 at the tip 114. In the depicted embodiment, the introducer 120 is an elongated extension from the tip 114. In the depicted embodiment, the introducer 120 is a narrow cylinder extending from the tip 114 at the distal end 106. As shown, the introducer 120 is also cannulated such that the introducer 120 has the outer surface 118 with the opening 116 to the channel 112 extending through the body 102. The introducer 120 allows for easier insertion of the surgical screw 100 because the introducer 120 can be used to align the surgical screw 100 at the desired bone hole location.

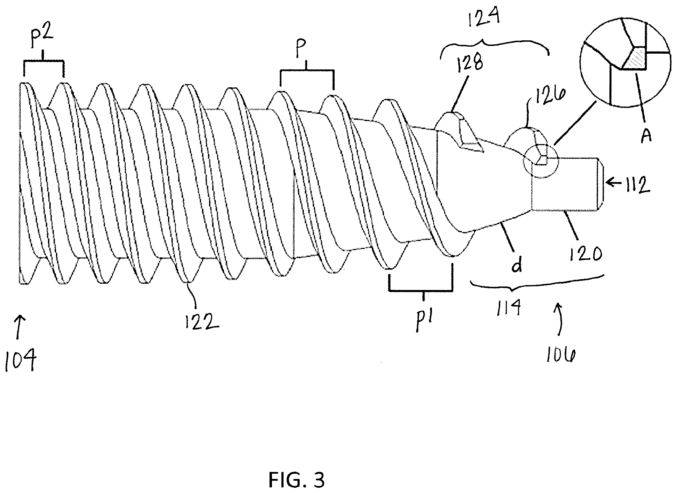

Referring now to FIG. 3, there is shown a side view schematic representation of the surgical screw 100, according to an embodiment. As shown in FIG. 3, the surgical screw 100 comprises a plurality of threads 122 along the length of the body 102. As with all conventional screws, adjacent threads 122 have a pitch p and root diameter d. However, the pitch p and therefore, the root diameter d of the depicted embodiment of the surgical screw 100 can be variable/non-constant (alternatively, the pitch can be variable and the root diameter can be constant or vice versa, and the pitch and/or the root diameter can be variable for a certain length of the body and constant for another length of the body). In other words, the threads 122 of the surgical screw 100 are not evenly spaced. In one embodiment, the pitch p increases from the proximal end 104 to the distal end 106, while the root diameter d increases from the distal end 106 to the proximal end 104. For example, as shown in FIG. 3, the pitch p1 at the distal end 106 is larger than the pitch p2 at the proximal end 104, and the root diameter at the distal end 106 is smaller than the root diameter at the proximal end 104.

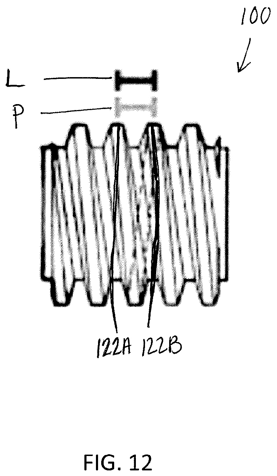

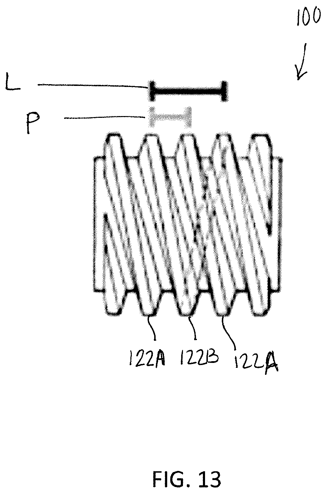

Still referring to FIG. 3, the threads 122 of the surgical screw 100 are dual start threads. Dual start threads are two threads opposed at 180 degrees. As shown in FIG. 3, the surgical screw 100 comprises a staggered start 124. In other words, a secondary thread 128 begins 180 degrees later than a primary thread 126. As shown in FIG. 13, the primary thread 122A has a lead L, which is greater than the pitch p of the threads 122 (122A, 122B) when the surgical screw 100 has a dual start or staggered start 124. The surgical screw 100 may also have a triple start or any additional multiple start (as should be understood by a person of ordinary skill in the art in conjunction with a review of this disclosure, not shown). Not claimed is a single start (FIG. 12) where the lead L and the pitch p are equal. In an embodiment, as shown in FIG. 3, the primary thread 126 has a reduced surface area A. The reduced surface area A reduces resistance and lowers torque during initial engagement with the bone.

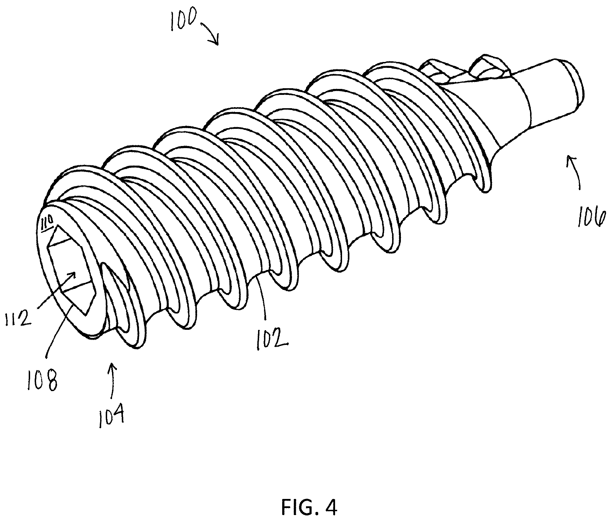

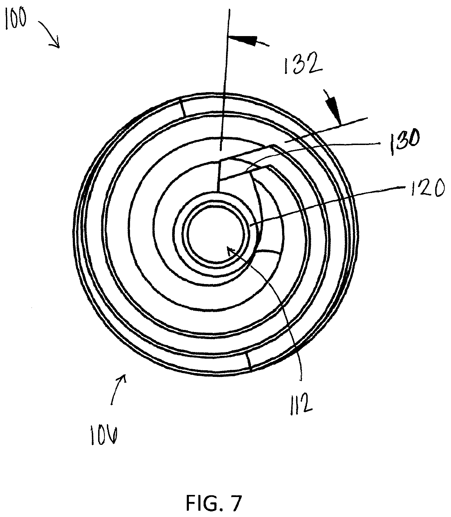

Turning now to FIGs. 4-11, there are shown various views schematic representations of surgical screw 100 according to alternative embodiments. First, in FIG. 4, there is shown a perspective view schematic representation of a surgical screw 100, according to an alternative embodiment. As with the surgical screw 100 in FIGs. 1-3, the surgical screw 100 in FIG. 4 comprises a body 102 extending between a proximal end 104 and a distal end
106 with a feature 108 on an outer surface 110 at the proximal end 104, which allows the surgical screw 100 to be torqued. As shown in FIG. 4, the surgical screw 100 is cannulated such that a channel 112 extends from the feature 108 at the outer surface 110 of the proximal end 104 through the body 102 to the distal end 106. As with the surgical screw 100 of FIGs. 1-3, the surgical screw 100 shown in FIGs. 4-11 can be composed of any suitable biocompatible material, such as titanium or magnesium, or bio-composite material, and can be manufactured according to common manufacturing methods, such as machining or injection molding, for example.

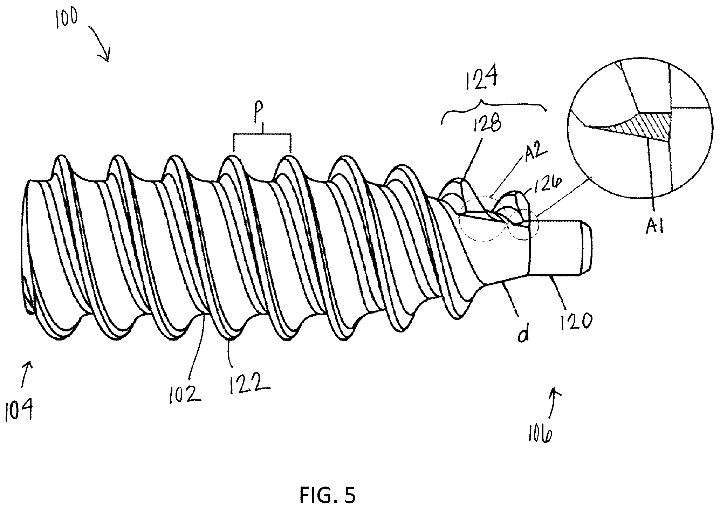

FIG. 5 shows a side view schematic representation of a surgical screw 100, according to an alternative embodiment. The surgical screw 100 shown in FIG. 5 has threads 122 extending along the body 102 of the screw 100. Adjacent threads 122 in the depicted embodiment have a constant (i.e., equal) pitch p. However, the surgical screw 100 may also have threads 122 with a variable pitch p1, p2, as shown in the embodiment in FIG. 3. The embodiment of the surgical screw 100 in FIG. 5 also has threads 122 with a root diameter d that is tapered from the proximal end 104 to the distal end 106. Specifically, the root diameter d decreases from the proximal end 104 to the distal end 106 (which can be a constant decrease in diameter in some embodiments, and a non-constant decrease in diameter in other embodiments).

Still referring to FIG. 5, the embodiment of the surgical screw 100 also includes a dual start thread (i.e., staggered start) 124. As stated above, the dual start thread 124 includes a primary thread 126, which is 180 degrees opposed from a secondary thread 128 such that the secondary thread 128 starts 180 degrees later (i.e., around the exterior of the body 102) than the primary thread 126. However, in other embodiments, the secondary thread 128 can start anywhere from a little as 5 degrees to as most as 360 degrees later than the primary thread 126. The primary thread 126 and secondary thread 128 have reduced surface areas A1, A2 to reduce resistance and lower torque during initial engagement with the bone.

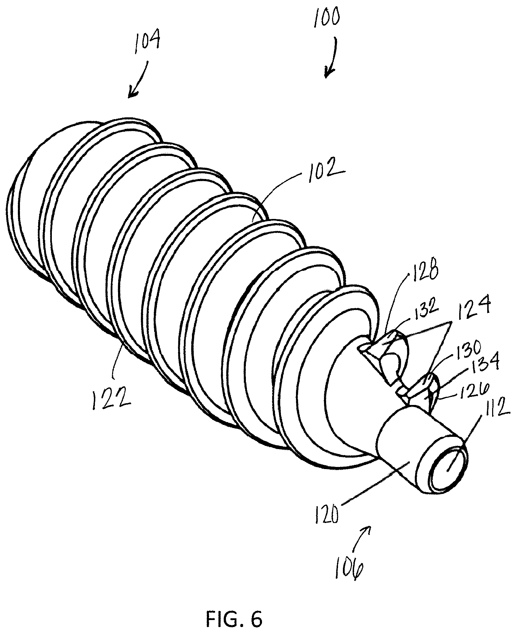

Turning now to FIGs. 6-7, there are shown distal end and distal perspective views schematic representations of a surgical screw 100, according to an alternative embodiment. In the depicted embodiment of the surgical screw 100, the primary thread 126 and the secondary thread 128 are chamfered. The chamfering removes a leading edge from the primary and secondary threads, further reducing the surface area A1, A2 thereof. As shown in FIG. 6, the chamfering creates a first sharp edge 130 on the primary thread 126 and a second sharp edge 132 on the secondary thread 128. The sharp edges 130, 132 allow for better engagement between the surgical screw 100 and bone. In the depicted embodiment, the distal portion 134 of the primary thread 126 is configured such that the surface area A1 of the primary thread 126 is further reduced as compared to the surface area A2 of the secondary thread 128. In additional embodiments, a radius is used in place of the chamfer when the surgical screw 100 is used for engagement with soft tissue as opposed to bone.

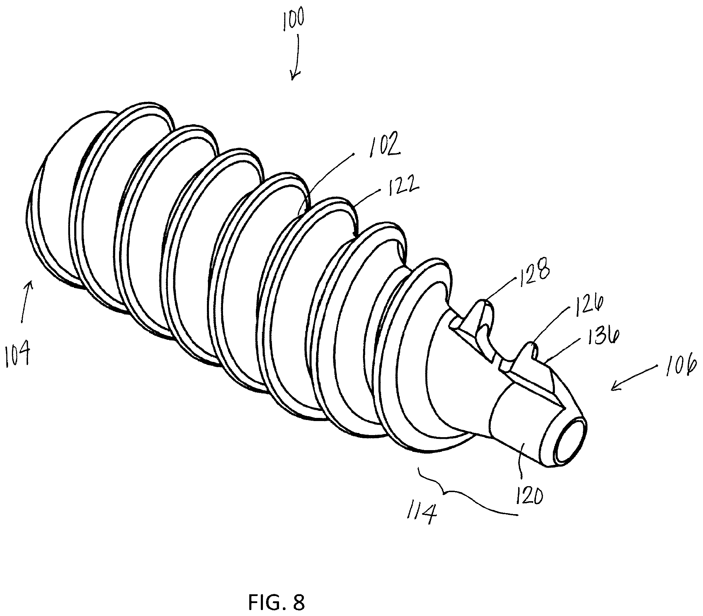

Referring now to FIG. 8, there is shown a distal perspective view schematic representation of a surgical screw 100, according to an embodiment. In the depicted embodiment, the surgical screw 100 includes a notching feature 136 at the distal end 106. In one embodiment, as shown in FIG. 8, the notching feature 136 extends from the introducer 120 at the distal end 106 of the surgical screw 100. In the depicted embodiment, the notching feature 136 is a notch that extends approximately tangentially from the introducer 120. The notching feature 136 can be malleted into the bone when the bone is hard. For example, a surgeon will align the introducer 120 at the desired bone hole location and mallet the proximal end 104 of the surgical screw 100 such that the introducer 120 and notching feature 136 engage the bone. In another embodiment, the notching feature 136 is used to find gaps between a primary bone and a graft when the surgical screw 100 is rotated. When the notching feature 136 finds a gap, it directs the primary thread 126 to the desired area and the surgical screw 100 can be rotated and installed without the need for malleting.

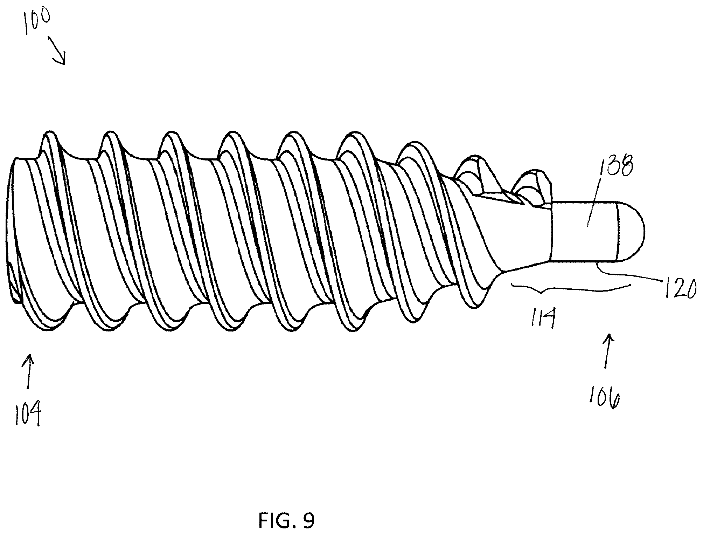

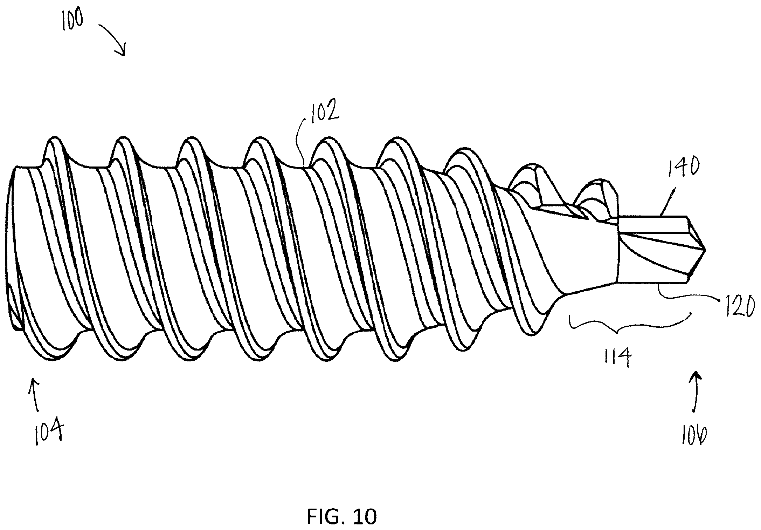

Turning now to FIGs. 9-10, there are shown side views schematic representations of a surgical screw 100, according to additional embodiments that do not fall within the scope of the invention as claimed. In the embodiment shown in FIGs. 9-10, the opening 116 to the channel 112 at the distal end 106 (introducer 120) of the surgical screw 100 is solid or otherwise replaced with a solid feature 138. The solid feature 138 may be rounded, as shown in FIG. 9, or the solid feature 138 may have any other suitable geometry. For example, the solid feature 138 may be a drill tip 140, as shown in FIG. 10. In the embodiments shown in FIGs. 9-10, the solid feature 138 is not cannulated. However, in alternative embodiments, the cannulation may also be completely removed (as in FIGs. 9-10) or only partially removed. In other words, the solid feature 138 can be either completely solid or only partially solid.

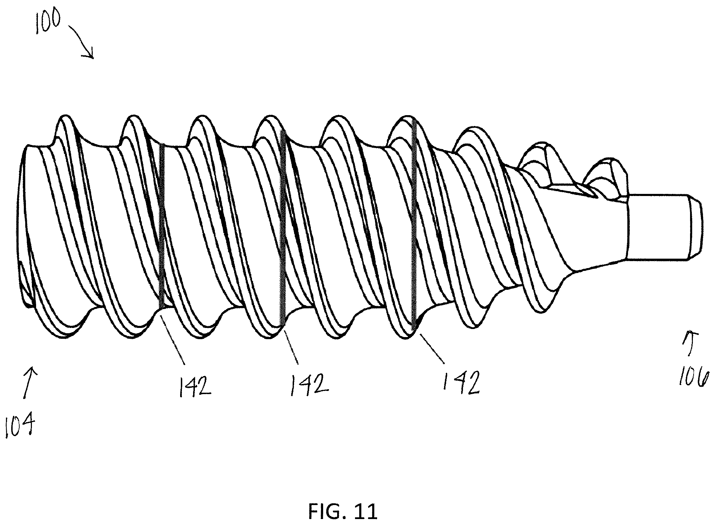

Referring now to FIG. 11, there is shown a side view schematic representation of a surgical screw 100, according to yet another embodiment. In the depicted embodiment, the surgical screw 100 comprises one or more exterior markings 142 along the body 102 between the proximal end 104 and the distal end 106. The exterior markings 142 of the surgical screw 100 in FIG. 11 are depth indicator markings 142. The depth indicator markings 142 are visible to a surgeon (or other user) during rotation and installation of the surgical screw 100. The depth indicator markings 142 allow the surgeon to determine the depth of the surgical screw 100 within the bone or soft tissue. In the depicted embodiment, the depth indicator markings 142 are circumferential markings extending around the exterior of the surgical screw 100. However, the depth indicator markings 142 can be placed anywhere along the exterior of the surgical screw 100 such that they are visible to the surgeon (or other user). As shown in FIG. 11, the depth indicator markings 142 can include multiple markings 142 spaced at equidistant intervals (e.g., 5 mm), a single depth marking, or multiple markings spaced at irregular intervals, as desired, and can be made from or coated with radiographic material.

All definitions, as defined and used herein, should be understood to control over dictionary definitions and/or ordinary meanings of the defined terms.

While various embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. It is to be understood that the foregoing embodiments are presented by way of example only and that, within the scope of the appended claims, embodiments may be practiced otherwise than as specifically described and claimed. Embodiments of the present disclosure are directed to each individual feature, system, article, material, kit, and/or method described herein. The scope of the invention is defined by the appended claims.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as, "has" and "having"), "include" (and any form of include, such as "includes" and "including"), and "contain" (any form of contain, such as "contains" and "containing") are open-ended linking verbs. As a result, a device that "comprises", "has", "includes" or "contains" one or more elements. Likewise, an element of a device that "comprises", "has", "includes" or "contains" one or more features possesses those one or more features, but is not limited to possessing only those one or more features. Furthermore, a device or structure that is configured in a certain way is configured in at least that way, but may also be configured in ways that are not listed.

The description of the present invention has been presented for purposes of illustration and description, but is not intended to be exhaustive or limited to the invention in the form disclosed. Many modifications and variations will be apparent to those of ordinary skill in the art without departing from the scope of the invention. The embodiment was chosen and described in order to best explain the principles of one or more aspects of the invention and the practical application, and to enable others of ordinary skill in the art to understand one or more aspects of the present invention for various embodiments with various modifications as are suited to the particular use contemplated. The scope of the invention is defined by the appended claims.

## Claims

1. A surgical screw (100), comprising:
a cannulated body (102) having a proximal end (104) and a distal end (106), the distal end (106) having a tapered tip region (114);
a plurality of threads (122) positioned around at least a portion of the exterior of the cannulated body (102), including a primary thread (126) and a secondary thread (128);
an introducer (120) extending distally from the tapered tip region (114); and
a channel (112) extending through the cannulated body (102) from the proximal end (104) to the distal end (106),
**characterised in that** the introducer (120) is a narrow cylinder having a constant diameter which is less than a diameter (d) of the tapered tip region (114), and the introducer (120) is cannulated such that the channel (112) extends from the proximal end (104) through the introducer (120).

2. The surgical screw (100) of any preceding claim, further comprising one or more exterior markings (142) on the cannulated body (102) indicating a depth.

3. The surgical screw (100) of any preceding claim, further comprising a notching feature (136) extending from the introducer (120), wherein the notching feature (136) preferably is a notch that extends approximately tangentially from the introducer (120).

4. The surgical screw (100) of any preceding claim, wherein the cannulated body (102) is composed of titanium.

5. The surgical screw (100) of any preceding claim, wherein a lead (L) of the primary thread (122A; 126) is greater than a pitch (p) of the plurality of threads (122);

6. The surgical screw (100) of claim 5, wherein each pair of adjacent threads of the plurality of threads (122) has a pitch (p) and a root diameter (d), and each pitch (p) is equal and each root diameter (d) is equal.

7. The surgical screw (100) of claim 5, wherein
the plurality of threads (122) include a first pair of adjacent threads and a second pair of adjacent threads, the first pair of adjacent threads having a first pitch (p1) and the second pair of adjacent threads having a second pitch (p2), wherein the first pitch (p1) is greater than the second pitch (p2), and
the first pair of adjacent threads have a first root diameter and the second pair of adjacent threads having a second root diameter, wherein the first root diameter is preferably greater than the second root diameter.

8. The surgical screw (100) of any preceding claim, wherein the primary thread (126) and the secondary thread (128) are opposed at an angle of 180 degrees.

9. The surgical screw (100) of any of claims 5 to 8, further comprising a notch (136) that extends approximately tangentially from the introducer (120).

## Patentansprüche

1. Chirurgische Schraube (100), umfassend:
einen kanülierten Körper (102) mit einem proximalen Ende (104) und einem distalen Ende (106), wobei das distale Ende (106) einen konischen Spitzenbereich (114) aufweist;
mehrere Gewinde (122), die um zumindest einen Teil des Äußeren des kanülierten Körpers (102) herum angeordnet sind, einschließlich eines Hauptgewindes (126) und eines Nebengewindes (128);
einen Einführer (120), der sich distal von dem konischen Spitzenbereich (114) erstreckt; und
einen Kanal (112), der sich von dem proximalen Ende (104) zu dem distalen Ende (106) durch den kanülierten Körper (102) erstreckt,
dadurc**h gekennzeichnet**, dass der Einführer (120) ein schmaler Zylinder mit einem konstanten Durchmesser ist, der kleiner als ein Durchmesser (d) des konischen Spitzenbereichs (114) ist, und der Einführer (120) kanüliert ist, so dass sich der Kanal (112) von dem proximalen Ende (104) durch den Einführer (120) erstreckt.

2. Chirurgische Schraube (100) nach einem vorhergehenden Anspruch, ferner umfassend eine oder mehrere Außenmarkierungen (142) auf dem kanülierten Körper (102), die eine Tiefe anzeigen.

3. Chirurgische Schraube (100) nach einem vorhergehenden Anspruch, ferner umfassend ein Kerbmerkmal (136), das sich von dem Einführer (120) erstreckt, wobei das Kerbmerkmal (136) vorzugsweise eine Kerbe ist, die sich ungefähr tangential von dem Einführer (120) erstreckt.

4. Chirurgische Schraube (100) nach einem vorhergehenden Anspruch, wobei der kanülierte Körper (102) aus Titan gebildet ist.

5. Chirurgische Schraube (100) nach einem vorhergehenden Anspruch, wobei eine Steigung (L) des Hauptgewindes (122A; 126) größer als eine Ganghöhe (p) der mehreren Gewinde (122) ist.

6. Chirurgische Schraube (100) nach Anspruch 5, wobei jedes Paar benachbarter Gewinde der mehreren Gewinde (122) eine Ganghöhe (p) und einen Kerndurchmesser (d) aufweist, und jede Ganghöhe (p) gleich ist und jeder Kerndurchmesser (d) gleich ist.

7. Chirurgische Schraube (100) nach Anspruch 5, wobei
die mehreren Gewinde (122) ein erstes Paar benachbarter Gewinde und ein zweites Paar benachbarter Gewinde umfassen, wobei das erste Paar benachbarter Gewinde eine erste Ganghöhe (p1) aufweist und das zweite Paar benachbarter Gewinde eine zweite Ganghöhe (p2) aufweist, wobei die erste Ganghöhe (p1) größer ist als die zweite Ganghöhe (p2), und
das erste Paar benachbarter Gewinde einen ersten Kerndurchmesser aufweist und das zweite Paar benachbarter Gewinde einen zweiten Kerndurchmesser aufweist, wobei der erste Kerndurchmesser vorzugsweise größer als der zweite Kerndurchmesser ist.

8. Chirurgische Schraube (100) nach einem vorhergehenden Anspruch, wobei das Hauptgewinde (126) und das Nebengewinde (128) in einem Winkel von 180 Grad gegenüberliegend angeordnet sind.

9. Chirurgische Schraube (100) nach einem der Ansprüche 5 bis 8, ferner umfassend eine Kerbe (136), die sich ungefähr tangential von dem Einführer (120) erstreckt.

## Revendications

1. Vis chirurgicale (100), comprenant :
un corps canulé (102) ayant une extrémité proximale (104) et une extrémité distale (106), l'extrémité distale (106) ayant une région de pointe effilée (114) ;
une pluralité de filets (122) positionnés autour d'au moins une partie de l'extérieur du corps canulé (102), incluant un filet principal (126) et un filet secondaire (128) ;
un introducteur (120) s'étendant distalement depuis la région de pointe effilée (114) ; et
un canal (112) s'étendant à travers le corps canulé (102) de l'extrémité proximale (104) à l'extrémité distale (106),
**caractérisée en ce que** l'introducteur (120) est un cylindre étroit ayant un diamètre constant qui est inférieur à un diamètre (d) de la région de pointe effilée (114), et l'introducteur (120) est canulé de telle sorte que le canal (112) s'étend depuis l'extrémité proximale (104) à travers l'introducteur (120).

2. Vis chirurgicale (100) selon une quelconque revendication précédente, comprenant en outre une ou plusieurs marques extérieures (142) sur le corps canulé (102) indiquant une profondeur.

3. Vis chirurgicale (100) selon une quelconque revendication précédente, comprenant en outre une caractéristique (136) d'entaillage s'étendant depuis l'introducteur (120), dans laquelle la caractéristique (136) d'entaillage est préférablement une entaille qui s'étend approximativement tangentiellement depuis l'introducteur (120).

4. Vis chirurgicale (100) selon une quelconque revendication précédente, dans laquelle le corps canulé (102) est composé de titane.

5. Vis chirurgicale (100) selon une quelconque revendication précédente, dans laquelle un pas d'entrée (L) du filet principal (122A ;126) est plus grand qu'un pas (p) de la pluralité de filets (122).

6. Vis chirurgicale (100) selon la revendication 5, dans laquelle chaque paire de filets adjacents parmi la pluralité de filets (122) a un pas (p) et un diamètre à fond de filet (d), et chaque pas (p) est égal et chaque diamètre à fond de filet (d) est égal.

7. Vis chirurgicale (100) selon la revendication 5, dans laquelle
la pluralité de filets (122) inclut une première paire de filets adjacents et une deuxième paire de filets adjacents, la première paire de filets adjacents ayant un premier pas (p1) et la deuxième paire de filets adjacents ayant un deuxième pas (p2), dans laquelle le premier pas (p1) est plus grand que le deuxième pas (p2), et
la première paire de filets adjacents a un premier diamètre à fond de filet et la deuxième paire de filets adjacents ayant un deuxième diamètre à fond de filet, dans laquelle le premier diamètre à fond de filet est préférablement plus grand que le deuxième diamètre à fond de filet.

8. Vis chirurgicale (100) selon une quelconque revendication précédente, dans laquelle le filet principal (126) et le filet secondaire (128) sont opposés à un angle de 180 degrés.

9. Vis chirurgicale (100) selon l'une quelconque des revendications 5 à 8, comprenant en outre une entaille (136) qui s'étend approximativement tangentiellement depuis l'introducteur (120).
